(19) Europäisches Patentamt
European Patent Office
Office européen des brevets

(11) **EP 2 950 919 B1**

(12) **EUROPÄISCHE PATENTSCHRIFT**

(45) Veröffentlichungstag und Bekanntmachung des Hinweises auf die Patenterteilung:
**01.05.2019 Patentblatt 2019/18**

(21) Anmeldenummer: **14703258.5**

(22) Anmeldetag: **28.01.2014**

(51) Int Cl.:
***B01F 15/00*** *(2006.01)*     ***B01F 1/00*** *(2006.01)*

(86) Internationale Anmeldenummer:
**PCT/EP2014/000215**

(87) Internationale Veröffentlichungsnummer:
**WO 2014/117927 (07.08.2014 Gazette 2014/32)**

(54) **VERFAHREN ZUR BEREITSTELLUNG EINES KONZENTRATS**

METHOD FOR PROVIDING A CONCENTRATE

PROCÉDÉ POUR PRÉPARER UN CONCENTRÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.01.2013 DE 102013001628**
**30.01.2013 US 201361758470 P**

(43) Veröffentlichungstag der Anmeldung:
**09.12.2015 Patentblatt 2015/50**

(73) Patentinhaber: **Fresenius Medical Care Deutschland GmbH**
**61352 Bad Homburg (DE)**

(72) Erfinder:
• **KOCH, Michael**
**verstorben (DE)**
• **EBERLEIN, Achim**
**97424 Schweinfurt (DE)**
• **NOACK, Joachim**
**97616 Bad Neustadt (DE)**

(74) Vertreter: **Herrmann, Uwe**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
**EP-A1- 0 556 098      EP-A1- 0 714 668**
**EP-A2- 0 548 537      DE-A1- 3 443 911**
**DE-U1- 9 418 915      DE-U1- 29 710 097**

# EP 2 950 919 B1

## Beschreibung

Die vorliegende Erfindung betrifft ein Verfahren zur Bereitstellung eines Konzentrats wenigstens eines gelösten Stoffes zur Herstellung einer Dialyselösung.

Bei einer Dialysebehandlung mit einer Online-Herstellung der Dialyselösung wird zur Bereitstellung des basischen Konzentrats üblicherweise ein Beutel mit festem Natriumbicarbonat verwendet.

Vor Behandlungsbeginn wird in diesen Beutel von der Dialysemaschine Wasser eingeleitet und so eine gesättigte Lösung des Natriumbicarbonates hergestellt. Die Überwachung des Füllstandes der Flüssigkeit bzw. des Fest-Flüssig-Gemisches in dem Beutel erfolgt bei aus dem Stand der Technik bekannten Geräten durch eine Druckmessung. Wird ein vorgegebener Druckwert unterschritten, wird in den Beutel Wasser nachgefüllt. Die entnommene Flüssigkeitsmenge wird somit durch frisches Wasser ersetzt, sobald der Druck in dem Beutel unter eine Schwelle absinkt, das heißt es erfolgt ein druckgesteuertes Nachfüllen des Wassers. Sobald das in dem Beutel befindliche Natriumbicarbonat aufgelöst ist, kann keine gesättigte Lösung mehr hergestellt werden und die Konzentration der Lösung im Beutel sinkt dementsprechend ab.

Unterschreitet die Konzentration einen bestimmten Wert, kann die notwendige Menge an Natriumbicarbonat durch die Exzentermembranpumpe oder ein sonstiges Förderorgan nicht mehr zur Herstellung einer Dialyselösung gefördert bzw. genutzt werden und es kommt zu einem Bicarbonatalarm.

Dies hat zur Folge, dass der Anwender den noch gefüllten Beutel von der Dialysemaschine abnehmen und einen neuen Beutel anhängen muss, der mit dem zu lösenden Feststoff gefüllt ist oder die Behandlung ist vorzeitig zu beenden.

Zu diesem Zeitpunkt ist der zuvor verwendete Beutel jedoch noch befüllt und zwar mit einer nicht ausreichend konzentrierten Bicarbonatlösung, die verworfen werden muss, obwohl die zu verwerfende Lösung noch Bicarbonat enthält.

Die EP 0 548 537 A2 bezieht ich auf eine Vorrichtung zur Herstellung einer Lösung aus einem Salz und Wasser. Das Salz befindet sich in einem Behälter, das Wasser stammt aus der Quelle. Stromabwärts des Behälters befindet sich eine Messzelle zur Ermittlung der Leitfähigkeit des Konzentrats, das aus dem Behälter abfließt. Stromabwärts des Mischpunktes zwischen diesem Konzentrat und dem Wasser befindet sich eine weitere Messzelle zur Messung der Leitfähigkeit der fertigen Dialyselösung. Diese hat die Aufgabe, die gesamte Einrichtung abzuschalten, wenn die Zusammensetzung der fertigen Dialyselösung nicht einem Sollwert entspricht.

Die EP 0 714 668 A1 bezieht sich auf eine Vorrichtung zur zentralen Herstellung eines gesättigten Salzkonzentrats. Dieses Konzentrat wird in eine Ringleitung geführt und kann daraus entnommen werden, um eine fertige medizinische Lösung herzustellen. Um sicherzustellen, dass kein Konzentrat mit einer unerwünschten Zusammensetzung verwendet wird, wird das Konzentrat mittels eines Ventils in den Abfluss verworfen wird.

In der Vorrichtung gemäß der DE 94 18 915 U1 wird eine Vorrichtung zur Herstellung einer Natriumbicarbonat-Lösung beschrieben. Mittels einer Leitfähigkeitsmesseinrichtung wird die Leitfähigkeit der hergestellten Lösung gemessen. Weicht diese von einem Sollwert ab, wird wird die gesamte Anlage abgestellt.

Die in der EP 0 556 098 A1 beschriebene Vorrichtung weist eine Mehrzahl von Behältern auf, die von Wasser durchströmt werden. Stromabwärts der Behälter befinden sich jeweils Leitfähigkeitsmesssonden. Deren Signal wird verwendet, um den Wasserzufluss so einzustellen, dass sich die gewünschte Konzentration ergibt.

Die DE 34 43 911 A1 offenbart eine Vorrichtung zur Herstellung einer Dialyselösung. Die Vorrichtung umfasst einen Behälter zur Herstellung der Lösung. Ein Abfluss aus dem Behälter wird nur dann freigegeben, wenn die Lösung eine bestimmte Leitfähigkeit aufweist.

Der vorliegenden Erfindung liegt daher die Aufgabe zugrunde, ein Verfahren der eingangs genannten Art dahingehend weiterzubilden, dass der Beutelinhalt optimal ausgenutzt werden kann, wodurch unnötiger Abfall vermieden wird.

Diese Aufgabe wird durch ein online-Verfahren mit den Merkmalen des Anspruchs 1 gelöst. Danach ist vorgesehen, dass das Verfahren die folgenden Schritte umfaßt:

- Bereitstellung wenigstens eines Behälters enthaltend wenigstens einen zu lösenden Feststoff;

- Zufuhr von Wasser in den Behälter zum Lösen des Feststoffes,

- Abfuhr des Konzentrats des gelösten Stoffes aus dem Behälter und Nachfüllen von Wasser,

- Messung der Konzentration des gelösten Stoffes oder eines mit dieser Konzentration korrelierten Parameters

- und vorübergehendes oder dauerhaftes Unterbinden des Nachfüllens von Wasser in den Behälter und Verwendung der nach dem Unterbinden des Nachfüllens von Wasser in dem Behältnis befindlichen Flüssigkeit zur Herstellung

2

der Dialyselösung, wenn der gemessene Konzentrationswert des gelösten Stoffes einen ersten Grenzwert unterschreitet bzw. der Wert des korrelierten Parameters einen ersten Grenzwert unter- oder überschreitet oder wenn die Änderung des Konzentrationswertes des gelösten Stoffes bzw. des Wertes des korrelierten Parameters einen ersten Grenzwert übersteigt.

Der vorliegenden Erfindung liegt somit der Gedanke zugrunde, das Nachfüllen mit Wasser dauerhaft oder vorübergehend einzustellen, wenn beispielsweise die Leitfähigkeit oder ein sonstiger Parameter, mittels dessen auf die Konzentration zurückgeschlossen werden kann, unter einen Grenzwert fällt oder diesen übersteigt. Wird das Nachfüllen von Wasser unterbunden, bevor die Konzentration im Beutel unter eine kritische Grenze fällt, so kann die im Beutel befindliche Bicarbonat-Restmenge bzw. die Menge des sonstigen gelösten Stoffes noch vollständig aufgebraucht bzw. für die Verwendung der Dialyselösung herangezogen werden.

Dies bringt nicht nur den Vorteil mit sich, dass der noch vorhandene gelöste Stoff in dem vorzugsweise als Beutel ausgebildeten Behälter weitgehend oder vollständig genutzt werden kann und Abfall vermieden wird, sondern auch den Vorteil, dass die Dialyse noch fortgesetzt werden kann, bis der Beutel vollständig entleert wurde.

Der Behälter wird vorzugsweise vollständig geleert, wenn nach dem Unterbinden des Nachfüllens von Wasser festgestellt wird, dass der Konzentrationswert des gelösten Stoffes nicht mehr ansteigt oder der Wert des mit der Konzentration korrelierten Parameters nicht mehr ansteigt oder nicht mehr fällt.

Gegebenenfalls kann somit die Verwendung eines zusätzlichen bzw. neuen Beutels oder sonstigen Behältnisses kurz vor Behandlungsende unter Umständen vermieden werden. Unter Umständen kann auch noch eine Online-Reinfusion durchgeführt werden und so ein zusätzlicher Beutel mit Kochsalzlösung eingespart werden.

Wesentlicher Gedanke der vorliegenden Erfindung ist es, ein Nachfüllen von Wasser in den Beutel zu unterbinden, wenn die Leitfähigkeit oder ein sonstiger mit der Konzentration des gelösten Stoffes in dem Beutel korrelierter Parameter einen Grenzwert unter- bzw. überschreitet. Bei diesem Parameter kann es sich beispielsweise um die Leitfähigkeit, die Transmission, die Absorption etc. handeln. Liegt die Leitfähigkeit, Konzentration, etc. in einem akzeptablen Bereich, verläuft die Befüllung und Entleerung des Behälters im Normalbetrieb, z. B. kann in diesem Fall das Nachfüllen mit Wasser druckgesteuert verlaufen, d. h. fällt der Druck unter einen Grenzwert, wird Wasser nachgefüllt, bis ein oberer Grenzwert für den Druck erreicht ist.

Bei Über- oder Unterschreiten des erfindungsgemäßen Grenzwertes für die Konzentration, Leitfähigkeit, etc. wird von dem Normalbetrieb in einen Betriebsmodus umgeschaltet, bei dem das Nachfüllen dauerhaft oder vorübergehend unterbunden wird.

Alternativ oder zusätzlich kann vorgesehen werden, dass z. B. auch eine zeitliche oder volumenbezogene oder sonstige Änderung der Leitfähigkeit oder eines sonstigen mit der Konzentration korrelierten Parameters erfasst wird und - wenn diese Änderung eine gewisse Grenze überschreitet - der Beutel oder das sonstige Behältnis für eine gewisse Zeit oder dauerhaft nicht mehr nachgefüllt wird.

So ist es beispielsweise denkbar, dass der Gradient der Leitfähigkeit über die Zeit gemessen wird. Überschreitet dieser einen bestimmten Grenzwert, kann vorgesehen sein, dass das Nachfüllen von Wasser zumindest vorübergehend unterbunden wird. Entsprechendes gilt z. B. für eine volumenbezogene Änderung. Ändert sich beispielsweise die Leitfähigkeit pro aus dem Behälter entnommener Volumeneinheit, kann ein Gradient aus der Leitfähigkeitsänderung und der Volumenänderung gebildet werden und darauf basierend entschieden werden, ob ein Nachfüllen von Wasser unterbleibt oder nicht.

Denkbar ist es, dass der Konzentrationswert nicht mehr ansteigt sondern bei der weiteren Zufuhr von Wasser aufgrund der eintretenden Verdünnung fällt, weil der Feststoff vollständig gelöst ist. Denkbar ist es jedoch auch, dass der Konzentrationswert wieder ansteigt oder sich der Gradient der Leitfähigkeit über die Zeit oder der Leitfähigkeitsänderung über das Volumen wieder ändert und zwar dann, wenn nur ein vorübergehendes Problem mit dem Auflösungsprozess des zu lösenden Feststoffes bestand. Kommt der Auflöseprozess wieder in Gang führt dies zu einem Anstieg der Konzentration, so dass wieder in den normalen Betriebsmodus zurückgekehrt werden kann, in dem ein Nachfüllen von Wasser z. B. druckgesteuert erfolgt.

Sollte die Konzentration auch nach Abschalten der Nachfüllroutine, d. h. nach Abschalten des Normalbetriebes nicht mehr ansteigen, kann davon ausgegangen werden, dass das Bicarbonat oder der wenigstens eine sonstige zu lösende Stoff vollständig gelöst wurde. In diesem Fall kann so lange Flüssigkeit aus dem Beutel oder sonstigem Behältnis gefördert werden und zur Herstellung der Dialyselösung verwendet werden, bis dieser/dieses leer ist. Dieser Zustand kann beispielsweise durch fehlende Flüssigkeit am Pegelsensor in der Bicarbonatkammer oder an dem fehlenden Überdruck in dem Behältnis erkannt werden.

Solange die Konzentration, die Leitfähigkeit der in dem Behältnis befindlichen bzw. aus diesem abgezogenen Flüssigkeit oder eine sonstige damit korrelierte Größe in einem akzeptablen Bereich liegt, kann die Steuerung bzw. Regelung der Zufuhr von Wasser zu dem Behältnis z. B. in Abhängigkeit vom Druck im Behältnis, in Abhängigkeit der Leitfähigkeit bzw. von deren zeitlichem Verlauf oder der Konzentration der entnommenen Lösung bzw. deren zeitlicher Verlauf, in Abhängigkeit vom entnommenen Volumen aus dem Behältnis, der benötigten Bicarbonatkonzentration für eine ausreichende Dosierung bzw. kritische Grenze, ab der keine ausreichende Dosierung mehr erfolgen kann, erfolgen.

Wird das Nachfüllen von Wasser ab einer bestimmten Grenze unterbunden, bringt dies den Vorteil mit sich, dass eine zu starke Verdünnung vermieden wird und dass das noch in dem Behältnis befindliche Konzentrat zur Herstellung der Dialyselösung verwendet werden kann.

In einer bevorzugten Ausgestaltung der Erfindung wird die Konzentration des gelösten Stoffes bzw. der korrelierte Parameter in dem aus dem Behälter ablaufenden Konzentrat gemessen. Alternativ dazu wäre auch eine Messung der Konzentration oder des sonstigen Parameters in dem Beutel selbst denkbar.

In weiterer Ausgestaltung der Erfindung ist vorgesehen, dass es sich bei der Änderung des Konzentrationswertes des gelösten Stoffes bzw. des Parameterwertes - wie oben ausgeführt - um die Änderung pro Zeiteinheit oder um die Änderung pro aus dem Behälter entnommener Volumeneinheit handelt. In diesen Fällen wird ein Gradient $dS/dt$ oder $dS/dV$ gebildet, wobei S für die Leitfähigkeit, t für die Zeit und V für das aus dem Behältnis entnommene Volumen stehen. Übersteigen diese Gradienten einen bestimmten Grenzwert, kann entschieden werden, dass das Nachfüllen von Wasser zumindest vorübergehend unterbleibt, d. h. der normale Betriebsmodus abgeschaltet wird. Kommt es beispielsweise aufgrund eines erneut einsetzenden Auflöseprozess zu einer Änderung des Gradienten oder zu einem Anstieg der Konzentration in einen gewünschten Bereich, kann vorgesehen sein, dass der Nachfüllprozess von Wasser und damit der Normalbetrieb wieder aktiviert wird.

In einer bevorzugten Ausgestaltung der Erfindung ist vorgesehen, dass der Behälter vorzugsweise vollständig geleert wird, wenn nach dem Unterbinden des Nachfüllens von Wasser festgestellt wird, dass der Konzentrationswert des gelösten Stoffes bzw. der Wert des mit der Konzentration korrelierten Parameters nicht mehr ansteigt bzw. keine Änderung des Trends (fallend/steigend) eintritt. Kommt es z. B. nicht mehr zu einem Anstieg der Konzentration kann darauf rückgeschlossen werden, dass kein aufzulösender Feststoff mehr vorliegt und dass das Zuführen weiteren Wassers nur noch zu einer weiteren Verdünnung und damit zu einer Konzentrationsminderung führen würde. In diesem Fall bleibt es dabei, das kein Wasser nachgefüllt wird und der Beutel vorzugsweise vollständig entleert wird.

Wie bereits oben ausgeführt ist es denkbar, dass das Nachfüllen mit Wasser wieder aktiviert wird, wenn festgestellt wird, dass der Konzentrationswert des gelösten Stoffes oder der Wert des mit der Konzentration korrelierten Parameters wieder in einem akzeptablen Bereich liegt, etwa weil lokale Auflöseprozesse wieder anspringen.

Denkbar ist es, dass der erste Grenzwert durch einen kritischen Grenzwert und einen Zuschlag gebildet wird.

Der minimal zulässige Wert für die Leitfähigkeit der aus dem Behältnis abströmenden Lösung bzw. des Konzentrats hängt von der gewünschten Sollkonzentration ab. Die benötigte Menge an Bicarbonat ergibt sich aus der Beziehung

$$c_{soll} \times V_{Bilanzkammer} < V_{max} \times c_{min}$$

wobei $V_{max}$ das maximale Fördervolumen der Bicarbonatpumpe, z. B. 2,2 ml ist. Geht man von einer Einstellung ($c_{soll}$) für Bicarbonat von 35 mmol Bicarbonat und 30 ml Bilanzkammervolumen aus, ergibt sich eine minimale Konzentration ($c_{min}$) im Behältnis bzw. Beutel von 477 mmol/l. Hieraus ergibt sich eine minimale Leitfähigkeit von ungefähr 29,5 mS/cm. Bei dieser minimalen Leitfähigkeit kann es sich um den kritischen Grenzwert handeln. Wird dieser unterschritten, ist die in dem Behältnis befindliche Lösung zur Herstellung einer Dialyselösung nicht mehr verwendbar, da sie zu stark verdünnt ist.

Um einen gewissen "Sicherheitsabstand" von dieser kritischen Grenze einzuhalten, kann es sinnvoll sein, dass der erste Grenzwert durch diesen kritischen Grenzwert und einen Zuschlag gebildet wird. Dies bedeutet, dass das Unterbinden des Nachfüllens nicht erst dann eingeleitet wird, wenn der genannte kritische Grenzwert erreicht ist, sondern dann, wenn der erste Grenzwert erreicht ist, der um einen Zuschlag von dem kritischen Grenzwert beabstandet ist. Denkbar ist es beispielsweise, eine 20%ige Reserve einzustellen, was in dem genannten Beispiel bedeuten würde, dass das Unterbinden des Nachfüllens bereits bei einem Wert der Leitfähigkeit von 34 mS/cm erfolgt. Steigt die Leitfähigkeit dann wieder an, so dass die Mindestkonzentration, d. h. die kritische Grenze um z. B. 30% überschritten wird, kann wieder nachgefüllt werden bzw. die übliche Nachfüllroutine wieder eingeleitet werden.

Somit ist es denkbar, dass das Nachfüllen mit Wasser wieder aktiviert wird, wenn der Konzentrationswert des gelösten Stoffes oder der Wert des mit der Konzentration korrelierten Parameters oder die Änderung $dS/dt$ oder $dS/dv$ etc. einen zweiten Grenzwert übersteigt.

Der zweite Grenzwert kann über dem ersten Grenzwert liegen, so dass das Verfahren mit einer gewissen Hysterese betrieben wird.

Weiterhin kann vorgesehen sein, dass der erste und/oder der zweite Grenzwert um einen prozentualen Betrag beispielsweise im Bereich von 20 - 30% von dem kritischen Wert beabstandet sind.

Weiterhin kann vorgesehen sein, dass es sich bei dem wenigstens einen gelösten Stoff ausschließlich oder auch um Bicarbonat handelt, vorzugsweise um Natriumbicarbonat. In bevorzugter Ausgestaltung der Erfindung ist vorgesehen, dass es sich bei dem gelösten Stoff um das sogenannte basische Konzentrat handelt, das im Rahmen der Herstellung einer Dialyselösung verwendet wird.

Bei dem Wasser kann es sich um RO (Revers-Osmose)-Wasser handeln.

An dieser Stelle wird darauf hingewiesen, dass der Begriff "Wasser" im Rahmen der vorliegenden Erfindung nicht nur RO-Wasser, sondern jedes beliebige Lösungsmittel umfasst.

Die vorliegende Erfindung betrifft des weiteren eine Dialysemaschine mit den Merkmalen des Anspruchs 11. Diese Dialysemaschine ist mit wenigstens einem Anschlussport ausgestattet, mit dem wenigstens ein Behälter, der einen zu lösenden Feststoff aufweist, konnektiert ist oder konnektierbar ist, wobei die Dialysemaschine des weiteren wenigstens eine Zufuhrleitung zur Zufuhr von Wasser in den Behälter und wenigstens eine Abfuhrleitung zur Abfuhr von Konzentrat aus dem Behälter aufweist und wobei die Dialysemaschine des weiteren wenigstens einen Sensor zur Messung der Konzentration oder zur Messung eines mit dieser korrelierten Parameters des aus dem Behälter entnommenen oder sich in diesem befindenden Konzentrats aufweist.

Erfindungsgemäß ist vorgesehen, dass die Dialysemaschine wenigstens eine Steuer- oder Regelungseinheit aufweist, die derart ausgebildet ist, dass sie die Bereitstellung eines Konzentrats eines gelösten Stoffes aus dem Behälter gemäß einem der oben genannten Ansprüche 1 bis 10 vornimmt. Dies bedeutet mit anderen Worten, dass die Steuer- oder Regelungseinheit derart ausgebildet ist, dass sie die Dialysemaschine nach diesem erfindungsgemäßen Verfahren betreiben kann.

Die Dialysemaschine weist somit Mittel zur Online-Herstellung einer Dialyselösung auf, die nach dem erfindungsgemäßen Verfahren betrieben werden.

Bei dem genannten Sensor der Dialysemaschine kann es sich um einen Leitfähigkeitssensor handeln. Grundsätzlich sind Konzentrationssensoren sowie auch jede beliebige andere Art von Sensoren von der Erfindung umfasst, deren Messwerte einen Rückschluss auf die Konzentration des wenigstens einen gelösten Stoffes ermöglichen.

Die Dialysemaschine kann wenigstens eine Einheit zur Erzeugung von RO-Wasser aufweisen oder wenigstens einen Anschluß aufweisen, durch den RO-Wasser der Dialysemaschine zuführbar ist. Bei dem dem Behälter zugeführten Wasser kann es sich dementsprechend um RO-Wasser handeln.

Denkbar ist es, dass die Maschine eine Hauptleitung aufweist und eine abgezweigte Nebenleitung, in der sich das wenigstens eine Behältnis befindet. Nach dem Durchströmen des Behältnisses wird das entsprechend gebildete Konzentrat mit dem RO-Wasser der Hauptleitung gemischt, so dass eine Dialyselösung in der gewünschten Konzentration vorliegt. Am Mischpunkt erfolgt somit eine Verdünnung des Konzentrates durch RO-Wasser.

Weiterhin kann vorgesehen sein, dass die Dialysemaschine Einstellmittel aufweist, mittels derer der erste Grenzwert und/oder der zweite Grenzwert und/oder der kritische Grenzwert und/oder der genannte Zuschlag zu dem kritischen Grenzwert durch einen Nutzer einstellbar ist.

Weiterhin kann vorgesehen sein, dass die Dialysemaschine Mittel zur Ausgabe eines Alarms aufweist, wenn der erste Grenzwert und/oder der zweite Grenzwert und/oder der kritische Grenzwert über- und/oder unterschritten wird.

Die Erfindung betrifft des Weiteren die Verwendung wenigstens eines Behälters, der mindestens einen zu lösenden Feststoff enthält in einem Verfahren und/oder in einer Dialysemaschine gemäß der vorliegenden Erfindung. Bei dem zu lösenden Feststoff kann es sich um Bicarbonat handeln bzw. der Feststoff kann aus Bicarbonat bestehen.

Weitere Einzelheiten und Vorteile der Erfindung werden anhand eines in der Zeichnung dargestellten Ausführungsbeispiels näher erläutert. Es zeigen:

Figur 1: einen zeitlichen Verlauf der Leitfähigkeit des aus einem Beutel ablaufenden Konzentrats über die Zeit,

Figur 2: Verlauf der Leitfähigkeit eines aus dem Beutel ablaufenden Konzentrats über die Zeit mit einem Einbruch der Bicarbonatkonzentration sowie mit einem Wechsel des Beutels und

Figur 3: eine vergrößerte Ansicht des Leitfähigkeitsverlaufs gemäß Figur 2 in dem Bereich großer Leitfähigkeitsänderungen.

Die Figuren sowie das im Folgenden beschriebene Ausführungsbeispiel beziehen sich auf das Lösen von Bicarbonat bzw. auf ein Bicarbonat-Konzentrat.

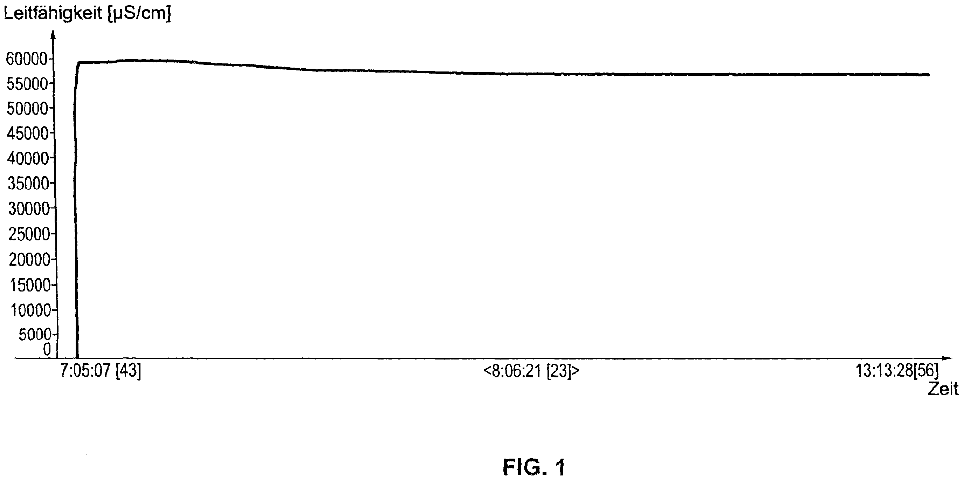

Aus Figur 1 ist ersichtlich, dass im Rahmen des Dialyseprozesses das aus dem Beutel ablaufende online-hergestellte Konzentrat eine im wesentlichen konstante Leitfähigkeit und damit Konzentration aufweist. Diese Konzentration kann der Sättigungskonzentration des oder der gelösten Stoffe entsprechen oder im wesentlichen dieser Sättigungskonzentration entsprechen. Figur 1 zeigt somit einen typischen Verlauf der Leitfähigkeit der aus einem Beutel entnommenen Flüssigkeit während einer Behandlung ohne den Beutelwechsel (temperaturkompensiert).

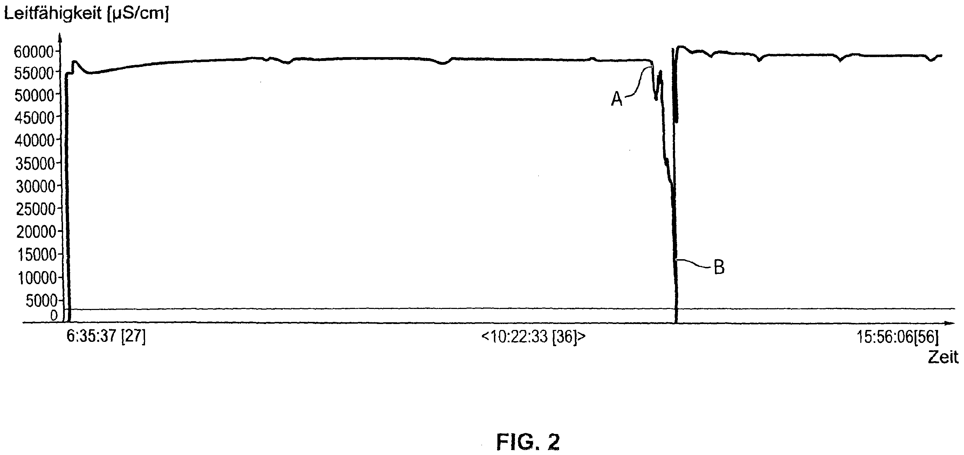

Figur 2 zeigt, dass ab einem bestimmten Zeitpunkt A ein Einbruch, das heißt ein starker Abfall der Leitfähigkeit und damit der Konzentration der aus dem Beutel abgezogenen Flüssigkeit eintritt. Dieser Einbruch der Leitfähigkeit erfolgt in dem hier dargestellten Beispiel 13 Minuten vor dem Bicarbonat- bzw. vor dem Leitfähigkeitsalarm zum Zeitpunkt B. Der Dialysatfluss betrug 940 ml/min. In der Zeit bis zum Bicarbonatalarm wurden ca. 400 ml aus dem Beutel entnommen.

Die Konzentration c im Beutel ergibt sich nach folgender Gleichung

$$c\,[mmol/l] = 10 + 11{,}52 \times LF\,(25°C)\,[mS/cm] + 0{,}156\,(LF\,(25°C)[mS/cm])^2.$$

Bei einem vollen Beutel liegt die Leitfähigkeit der Lösung wie aus Figur 2 ersichtlich bei etwa 57 mS/cm. Dies entspricht einer Konzentration von ca. 1173 mmol/l.

Wie bereits oben ausgeführt, hängt der minimal zulässige Wert für die Leitfähigkeit von der gewünschten Sollkonzentration ab. In dem oben genannten Beispiel ergibt sich eine minimale Leitfähigkeit von 29,5 mS/cm. Dies passt gut zum Auftreten des Bicarbonatalarms gemäß Figur 2. Dieser Wert wurde in dem hier dargestellten Ausführungsbeispiel 40 s vor der Alarmausgabe B unterschritten.

In dem in Figur 2 dargestellten Ausführungsbeispiel wurde zum Zeitpunkt B, das heißt zum Zeitpunkt des Auftreten des Bicarbonatalarms der Beutel gewechselt, das heißt der volle Beutel abgenommen und ein neuer Beutel mit frischem Feststoff an die Dialysemaschine angehängt. Dieser wurde sodann mit Wasser befüllt und es stellte sich erneut eine Leitfähigkeit im Bereich von ca. 57 mS/cm ein, wie dies aus Figur 2 hervorgeht.

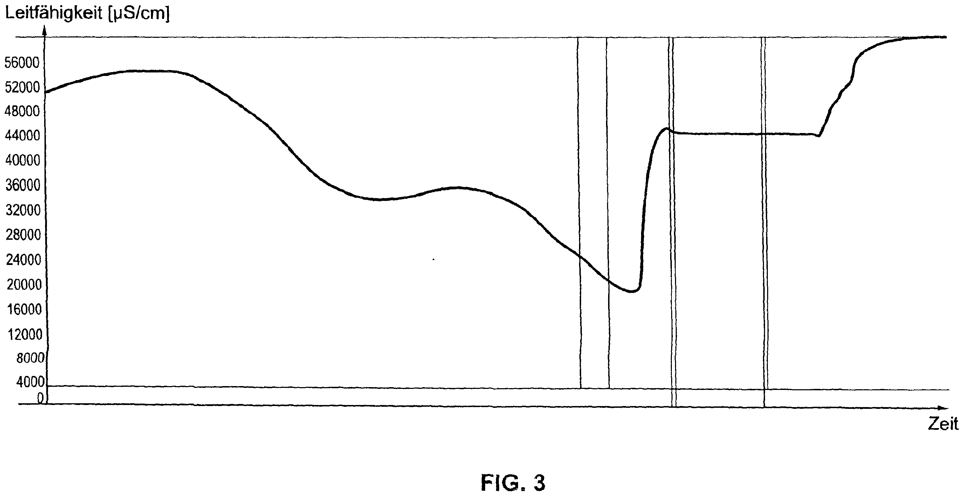

Figur 3 zeigt in vergrößerter Darstellung den Zeitabschnitt des Abfalls der Leitfähigkeit gemäß Figur 2.

Wie dies aus Figur 3 hervorgeht, wurde in dem hier dargestellten Ausführungsbeispiel der Beutel bei einem Leitfähigkeitswert von ca. 20 mS/cm ausgewechselt. Der Beutel mit dieser Konzentration wurde verworfen und ein neuer Beutel verwendet.

Erfindungsgemäß ist es nun möglich, ein zu starkes Abfallen der Konzentration wie in Figur 3 dargestellt dadurch zu verhindern, dass ab einem bestimmten Grenzwert der Leitfähigkeit ein Nachfüllen von Wasser unterbleibt. Dies führt dazu, dass die Konzentration in dem Beutel auf einem gewissen Mindestniveau bleibt, im konkreten Beispiel könnte beispielsweise ein Wert von 34 mS/cm als erster Grenzwert sinnvoll sein.

Steigt die Leitfähigkeit dann wieder an, weil Auflösungsprozesse wieder einsetzen, kann wieder nachgefüllt werden bzw. in den Nachfüllmodus umgeschaltet werden. Dies kann beispielsweise dann der Fall sein, wenn die Mindestkonzentration, das heißt der kritische Grenzwert z. B. um 30% überschritten wird.

Insgesamt ist festzustellen, dass durch die vorliegende Erfindung Abfall vermiedenwerden kann und je nach insgesamt erforderlicher Behandlungsdauer darüber hinaus der Vorteil erreicht werden kann, dass gegebenenfalls das Anhängen eines neuen zu lösenden Feststoff enthaltenden Beutels bzw. Behältnisses an das Gerät vermieden werden kann.

So kann die Dialysebehandlung noch fortgesetzt werden, bis der Beutel vollständig entleert wurde. Hierdurch wird das Anhängen eines weiteren Beutels kurz vor Behandlungsende unter Umständen unterbunden bzw. vermieden bzw. es kann ggf. eine Online-Reinfusion durchgeführt werden.

**Patentansprüche**

1. Verfahren zur online-Bereitstellung eines Konzentrats eines gelösten Stoffes in einer Dialysemaschine und zur online-Herstellung von einer Dialyselösung in einer Dialysemaschine, wobei das Verfahren die folgenden Schritte umfasst:

   Bereitstellung eines Behälters mit einem zu lösenden Feststoff;
   Zufuhr von Wasser in den Behälter zum Lösen des Feststoffes;
   Abfuhr des Konzentrats des gelösten Stoffes aus dem Behälter und Nachfüllen von Wasser,
   Messung der Konzentration des gelösten Stoffes oder eines mit der Konzentration korrelierten Parameters und Unterbinden des Nachfüllens von Wasser, wenn der gemessene Konzentrationswert des gelösten Stoffes bzw. der gemessene Parameterwert einen ersten Grenzwert unterschreitet oder eine Änderung des Konzentrationswerts bzw. des Parameterwerts einen ersten Grenzwert übersteigt,
   **gekennzeichnet durch**
   Verwendung der nach dem Unterbinden des Nachfüllens von Wasser in dem Behältnis befindlichen Flüssigkeit zur Herstellung der Dialyselösung.

2. Verfahren nach Anspruch 1, **dadurch gekennzeichnet, dass** die Konzentration des gelösten Stoffes oder der Parameter in dem aus dem Behälter ablaufenden Konzentrat gemessen wird.

3. Verfahren nach Anspruch 1 oder 2, **dadurch gekennzeichnet, dass** das Unterbinden des Nachfüllens von Wasser und die Verwendung der nach dem Unterbinden des Nachfüllens von Wasser in dem Behältnis befindlichen Flüssigkeit zur Herstellung der Dialyselösung erfolgt, wenn die Änderung des Konzentrationswertes des gelösten Stoffes bzw. des Wertes des Parameters einen ersten Grenzwert übersteigt, wobei es sich bei der Änderung des Konzentrationswertes des gelösten Stoffes bzw. des Parameterwertes um die Änderung pro Zeiteinheit oder um die Änderung pro aus dem Behälter entnommener Volumeneinheit handelt.

4. Verfahren nach einem der vorhergehenden Ansprüche, **dadurch gekennzeichnet, dass** der Behälter geleert wird, wenn nach dem Unterbinden des Nachfüllens von Wasser festgestellt wird, dass der Konzentrationswert des gelösten Stoffes nicht mehr ansteigt oder der Wert des mit der Konzentration korrelierten Parameters nicht mehr ansteigt oder nicht mehr fällt.

5. Verfahren nach einem der vorhergehenden Ansprüche, **dadurch gekennzeichnet, dass** es sich bei dem Wasser um RO-Wasser handelt.

6. Verfahren nach einem der vorhergehenden Ansprüche, **dadurch gekennzeichnet, dass** der erste Grenzwert durch einen kritischen Grenzwert und einen Zuschlag gebildet wird.

7. Verfahren nach einem der Ansprüche 5 oder 6, **dadurch gekennzeichnet, dass** das Nachfüllen mit Wasser wieder aktiviert wird, wenn der Konzentrationswert des gelösten Stoffes einen zweiten Grenzwert übersteigt oder der Wert des mit der Konzentration korrelierten Parameters einen zweiten Grenzwert übersteigt oder unterschreitet.

8. Verfahren nach Anspruch 7, **dadurch gekennzeichnet, dass** der zweite Grenzwert über oder unter dem ersten Grenzwert liegt oder diesem entspricht.

9. Verfahren nach einem der Ansprüche 6 bis 8, **dadurch gekennzeichnet, dass** der erste und/oder der zweite Grenzwert um einen prozentualen Betrag über oder unter dem kritischen Grenzwert liegen.

10. Verfahren nach einem der vorhergehenden Ansprüche, **dadurch gekennzeichnet, dass** es sich bei dem gelösten Stoff ausschließlich oder auch um Bicarbonat handelt, vorzugsweise um Natrium-Bicarbonat handelt.

11. Dialysemaschine mit
einem Anschlussport mit dem ein Behälter, der einen zu lösenden Feststoff aufweist, konnektiert ist,
einer Zufuhrleitung zur Zufuhr von Wasser in den Behälter,
einer Abfuhrleitung zur Abfuhr von Konzentrat aus dem Behälter, und
einem Sensor zur Messung der Konzentration oder zur Messung eines mit dieser korrelierten Parameters des aus dem Behälter entnommenen Konzentrats,
**dadurch gekennzeichnet, dass**
die Dialysemaschine eine Steuer- oder Regelungseinheit aufweist, die derart ausgebildet ist, dass sie die Bereitstellung eines Konzentrats eines-gelösten Stoffes aus dem Behälter gemäß einem der Ansprüche 1 bis 10 vornimmt.

12. Dialysemaschine nach Anspruch 11, **dadurch gekennzeichnet, dass** es sich bei dem Sensor um einen Leitfähigkeitssensor handelt.

13. Dialysemaschine nach Anspruch 11 oder 12, **dadurch gekennzeichnet, dass** die Dialysemaschine wenigstens eine Einheit zur Erzeugung von RO-Wasser aufweist oder wenigstens einen Anschluß aufweist, durch den RO-Wasser der Dialysemaschine zuführbar ist und dass es sich bei dem dem Behälter zugeführten Wasser um RO-Wasser handelt.

14. Dialysemaschine nach einem der Ansprüche 11 bis 13, **dadurch gekennzeichnet, dass** die Dialysemaschine Einstellmittel aufweist, mittels derer mindestens einer der folgenden Werte durch einen Nutzer einstellbar ist: der erste Grenzwert, der zweite Grenzwert, der kritische Grenzwert, der Zuschlag zu dem kritischen Grenzwert.

15. Dialysemaschine nach einem der Ansprüche 11 bis 14, **dadurch gekennzeichnet, dass** die Dialysemaschine Mittel zur Ausgabe eines Alarms aufweist, wenn mindestens einer der folgenden Werte über- oder unterschritten wird: der erste Grenzwert, der zweite Grenzwert, der kritische Grenzwert.

**Claims**

1. A method for the online provision of a concentrate of a dissolved substance in a dialysis machine and for the online production of a dialysis solution in a dialysis machine, the method comprising the following steps:

   providing a container containing a solid to be dissolved;
   supplying water into the container for dissolving the solid;
   draining the concentrate of the dissolved substance from the container and topping up with water;
   measuring the concentration of the dissolved substance or of a parameter correlated with this concentration;
   and suppressing the topping up with water when the measured concentration value of the dissolved substance or the measured value of the parameter falls below a first limit value or when a change in the concentration value or of the value of the parameter exceeds a first limit value,
   **characterized by**
   using the liquid present in the container after suppressing the topping up with water for the production of the dialysis solution.

2. A method in accordance with claim 1, **characterized in that** the concentration of the dissolved substance or the parameter in the concentrate running out of the container is measured.

3. A method in accordance with claim 1 or claim 2, **characterized in that** suppressing the topping up with water and using the liquid present in the container after suppressing the topping up with water for the production of the dialysis solution take place when the change of the concentration value of the dissolved substance or of the parameter value exceeds a first limit value, with the change of the concentration value of the dissolved substance or of the parameter value being the change per time unit or the change per volume unit taken from the container.

4. A method in accordance with one of the preceding claims, **characterized in that** the container is emptied when it is found after the suppression of the topping up with water that the concentration value of the dissolved substance is no longer increasing or the value of the parameter correlated with the concentration is no longer increasing or is no longer falling.

5. A method in accordance with one of the preceding claims, **characterized in that** the water is RO water.

6. A method in accordance with one of the preceding claims, **characterized in that** the first limit value is formed by a critical limit value and a supplement.

7. A method in accordance with one of the claims 5 or 6, **characterized in that** the topping up with water is activated again when the concentration value of the dissolved substance exceeds a second limit value or when the value of the parameter correlated with the concentration exceeds or falls below a second limit value.

8. A method in accordance with claim 7, **characterized in that** the second limit value lies above or below the first limit value or corresponds thereto.

9. A method in accordance with one of the claims 6 to 8, **characterized in that** the first and/or second limit value lies above or below the critical limit value by a percentage amount.

10. A method in accordance with one of the preceding claims, **characterized in that** the dissolved substance is only bicarbonate or also bicarbonate, preferably sodium bicarbonate.

11. A dialysis machine comprising
    a connector port to which a container having a solid to be dissolved is connected;
    a supply line for the supply of water into the container;
    a removal line for the removal of concentrate from the container; and
    a sensor for measuring the concentration or for measuring a parameter correlated therewith of the concentrate removed from the container,
    **characterized in that**
    the dialysis machine has a control or regulation unit which is configured such that it carries out the provision of a concentrate of a dissolved substance from the container in accordance with one of the claims 1 to 10.

**12.** A dialysis machine in accordance with claim 11, **characterized in that** the sensor is a conductivity sensor.

**13.** A dialysis machine in accordance with claim 11 or claim 12, **characterized in that** the dialysis machine has at least one unit for producing RO water or has at least one connector through which RO water can be supplied to the dialysis machine; and **in that** the water supplied to the container is RO water.

**14.** A dialysis machine in accordance with one of the claims claim 11 to 13, **characterized in that** the dialysis machine has setting means by means of which at least one of the following values can be set by the user: the first limit value, the second limit value, the critical limit value, the supplement to the critical limit value.

**15.** A dialysis machine in accordance with one of the claims claim 11 to 14, **characterized in that** the dialysis machine has means for emitting an alarm when at least one of the following values is/are exceeded or fallen below: the first limit value, the second limit value, the critical limit value.

**Revendications**

**1.** Procédé de préparation en ligne d'un concentré d'une substance dissoute dans une machine de dialyse et pour la fabrication en ligne d'une solution de dialyse dans une machine de dialyse, le procédé comprenant les étapes suivantes :

mise à disposition d'un récipient avec une substance solide à dissoudre ;
amenée d'eau dans le récipient pour dissoudre la substance solide ;
évacuation du concentré de la substance dissoute hors du récipient et ajout d'eau,
mesure de la concentration de la substance dissoute ou d'un paramètre en corrélation avec la concentration et arrêt de l'ajout d'eau quand la valeur de concentration mesurée de la substance dissoute ou la valeur mesurée du paramètre devient inférieure à une première valeur limite ou qu'une modification de la valeur de concentration ou de la valeur du paramètre dépasse une première valeur limite,
**caractérisé par**
l'utilisation du liquide se trouvant dans le récipient après l'arrêt de l'ajout d'eau pour la fabrication de la solution de dialyse.

**2.** Procédé selon la revendication 1, **caractérisé en ce que** la concentration de la substance dissoute ou le paramètre est mesuré(e) dans le concentré s'écoulant du récipient.

**3.** Procédé selon la revendication 1 ou 2, **caractérisé en ce que** l'arrêt de l'ajout d'eau et l'utilisation du liquide se trouvant dans le récipient après l'arrêt de l'ajout d'eau pour la fabrication de la solution de dialyse sont effectués quand la modification de la valeur de concentration de la substance dissoute ou de la valeur du paramètre dépasse une première valeur limite, la modification de la valeur de concentration de la substance dissoute ou de la valeur du paramètre étant la modification par unité de temps ou la modification par unité de volume prélevée du récipient.

**4.** Procédé selon l'une des revendications précédentes, **caractérisé en ce que** le récipient est vidé quand il est constaté après l'arrêt de l'ajout d'eau que la valeur de concentration de la substance dissoute n'augmente plus ou que la valeur du paramètre en corrélation avec la concentration n'augmente plus ou ne diminue plus.

**5.** Procédé selon l'une des revendications précédentes, **caractérisé en ce que** l'eau est de l'eau traitée par osmose inverse.

**6.** Procédé selon l'une des revendications précédentes, **caractérisé en ce que** la première valeur limite est formée par une valeur limite critique et un supplément.

**7.** Procédé selon l'une des revendications 5 ou 6, **caractérisé en ce que** l'ajout d'eau est réactivé quand la valeur de concentration de la substance dissoute dépasse une seconde valeur limite ou que la valeur du paramètre en corrélation avec la concentration dépasse ou devient inférieure à une seconde valeur limite.

**8.** Procédé selon la revendication 7, **caractérisé en ce que** la seconde valeur limite est supérieure ou inférieure à la première valeur limite ou lui correspond.

9. Procédé selon l'une des revendications 6 à 8, **caractérisé en ce que** la première et/ou la seconde valeur limite sont supérieures ou inférieures à la valeur limite critique selon un pourcentage.

10. Procédé selon l'une des revendications précédentes, **caractérisé en ce que** la substance dissoute est uniquement ou également du bicarbonate, de préférence du bicarbonate de sodium.

11. Machine de dialyse comprenant
un port de connexion au moyen duquel un récipient, qui comporte une substance solide à dissoudre, est connecté,
une conduite d'amenée pour amener de l'eau dans le récipient,
une conduite d'évacuation pour évacuer du concentré du récipient, et
un capteur pour mesurer la concentration ou pour mesurer un paramètre en corrélation avec celle-ci du concentré prélevé du récipient,
**caractérisée en ce que**
elle comporte une unité de commande ou de régulation, qui est conçue de telle manière qu'elle effectue la préparation d'un concentré d'une substance dissoute provenant du récipient selon l'une des revendications 1 à 10.

12. Machine de dialyse selon la revendication 11, **caractérisée en ce que** le capteur est un capteur de conductivité.

13. Machine de dialyse selon la revendication 11 ou 12, **caractérisée en ce qu'**elle comporte au moins une unité destinée à générer de l'eau traitée par osmose inverse ou au moins un raccordement, par lequel de l'eau traitée par osmose inverse peut être amenée dans la machine de dialyse et **en ce que** l'eau amenée dans le récipient est de l'eau traitée par osmose inverse.

14. Machine de dialyse selon l'une des revendications 11 à 13, **caractérisée en ce qu'**elle comporte des moyens de réglage, au moyen desquels au moins une des valeurs suivantes peut être réglée par un utilisateur : la première valeur limite, la seconde valeur limite, la valeur limite critique, le supplément à la valeur limite critique.

15. Machine de dialyse selon l'une des revendications 11 à 14, **caractérisée en ce qu'**elle comporte des moyens destinés à émettre une alarme en cas de passage au-dessus ou au-dessous d'au moins une des valeurs suivantes : la première valeur limite, la seconde valeur limite, la valeur limite critique.

FIG. 1

EP 2 950 919 B1

FIG. 2

FIG. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0548537 A2
- EP 0714668 A1
- DE 9418915 U1
- EP 0556098 A1
- DE 3443911 A1